# EUROPÄISCHE PATENTANMELDUNG

(11) **EP 0 808 627 A2**
(43) Veröffentlichungstag der Anmeldung: **26.11.1997**
(21) Anmeldenummer: 97107624.5
(22) Anmeldetag: 09.05.1997
(51) Int. Cl.: A61K 31/47, A61K 31/495, A61K 31/50, A61K 31/505, A61K 31/535, A61K 31/54

(54) **Verwendung nicht-peptidischer Bradykinin-Antagonisten zur Behandlung und Prävention von chronisch-fibrogenetischen Lebererkrankungen, akuten Lebererkrankungen und den damit verbundenen Komplikationen**

(30) Priorität: 22.05.1996 DE 19620509; 08.08.1996 DE 19632042; 25.09.1996 DE 19639303
(71) Anmelder: HOECHST AKTIENGESELLSCHAFT, 65929 Frankfurt am Main (DE)
(72) Erfinder: Heitsch, Holger, Dr., 55252 Mainz-Kastel (DE); Wagner, Adalbert, Dr., 86368 Gersthofen (DE); Wirth, Klaus, Dr., 65830 Kriftel (DE); Hropot, Max, Dr., 65439 Flörsheim (DE); Bickel, Martin, Dr., 61348 Bad Homburg (DE)

(57) **Zusammenfassung**

Die Erfindung betrifft die Verwendung von nicht-peptidischen Bradykinin-Antagonisten zur Herstellung von Arzneimitteln zur Behandlung von chronischfibrogenetischen Lebererkrankungen (Leberzirrhose und Leberfibrose), akuten Lebererkrankungen und zur Prävention von Komplikationen, insbesondere zur Prophylaxe bzw. Behandlung der portalen Hypertonie, Dekompensationserscheinungen wie Aszites, Ödembildung, hepatorenales Syndrom, hypertensive Gastro- und Colopathie, Splenomegalie sowie Blutungskomplikationen im Gastrointestinaltrakt durch portale Hypertonie, Kollateralkreislauf und Hyperämie und eine Kardiopathie als Folge einer chronisch hyperdynamen Kreislaufsituation und deren Folgen.

## Beschreibung

Bradykinin und verwandte Peptide sind potente, Entzündungen und Schmerz erzeugende und vasoaktive, körpereigene Substanzen. Aus EP-A 622 361, US 5,212,182, US 5,216,165, US 5,438,064 und WO 9604251 sind substituierte, annellierte Heterobicyclen und ihre Verwendung als Bradykinin-Rezeptorantagonisten und ihre Verwendung als Mittel zur Bekämpfung von Zuständen, die durch Bradykinin vermittelt, ausgelöst oder unterstützt werden, bekannt.

Überraschenderweise wurde nun gefunden, daß nicht-peptidische Bradykinin-Antagonisten dieses Strukturtyps darüber hinaus geeignete Mittel zur Behandlung von chronisch fibrogenetischen Lebererkrankungen (Leberzirrhose und Leberfibrose), akuten Lebererkrankungen und zur Prävention von Komplikationen, insbesondere zur Prophylaxe bzw. Behandlung der portalen Hypertonie, Dekomenpsationserscheinungen wie Aszites, Ödembildung, hepatorenales Syndrom, hypertensive Gastro- und Colopathie, Splenomegalie sowie Blutungskomplikationen im Gastrointestinaltrakt durch portale Hypertonie, Kollateralkreislauf und Hyperämie und eine Kardiopathie als Folge einer chronisch hyperdynamen Kreislaufsituation und deren Folgen, sind.

Als Verbindungen eignen sich nicht-peptidische Bradykinin-Antagonisten, die im Modell der CCl₄-induzierten Leberfibrose an der Ratte einen natriuretischen und diuretischen Effekt zeigen.

Geeignete nicht-peptidische Bradykinin-Antagonisten sind unter anderem die Verbindungen der Formel (I) 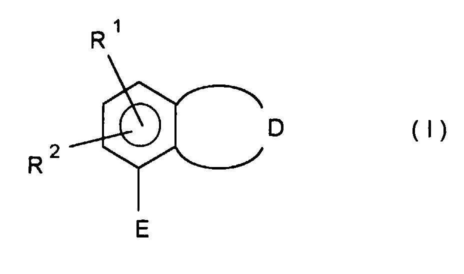 in welcher die Symbole folgende Bedeutung haben:
- D: 1. ein Rest der Formel (II): 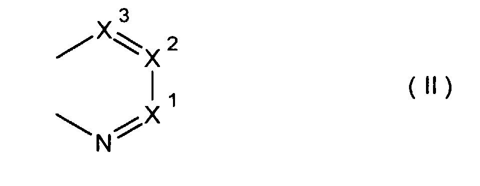
2. ein Rest der Formeln (III) bis (VI): 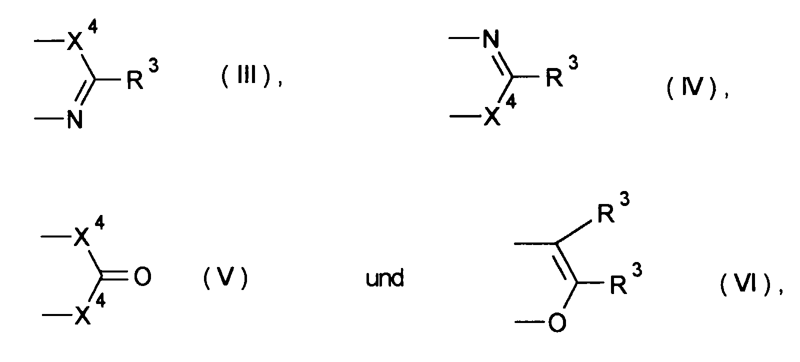
- E: 1. ein Rest der Formel (VII): 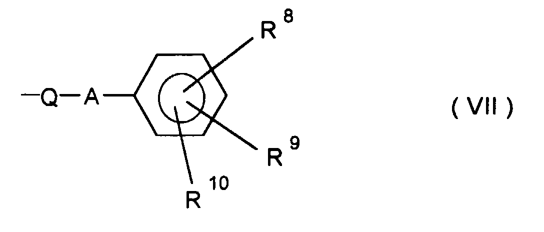
2. Wasserstoff, Halogen, (C₁-C₄)-Alkyl, (C₁-C₄)-Alkylthio, (C₁-C₄)-Alkoxy, wobei in den letzten 3 Resten 1 oder mehrere Wasserstoffatome durch Fluor ersetzt sein können;
- X¹: Stickstoff oder C-R⁴ ;
- X²: Stickstoff oder C-R⁵ ;
- X³: Stickstoff oder C-R⁶ ;
- X⁴: Sauerstoff, Stickstoff oder N-R⁷ ;
- R¹,R²: sind gleich oder verschieden und stehen für Wasserstoff, Halogen, (C₁-C₄)-Alkyl oder (C₁-C₄)-Alkoxy;
- R³: Wasserstoff, Halogen, (C₁-C₆)-Alkyl, (C₆-C₁₂)-Aryl, (C₁-C₃)-Alkyl-(C₆-C₁₂)-Aryl, (C₃-C₅)-Alkenyl, (C₁-C₄)-Alkoxy, CO₂R¹¹;
- R⁴: Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, das gegebenenfalls substituiert ist mit Hydroxy, (C₁ -C₄)-Alkoxy, Amino oder (C₁-C₄)-Alkylamino, oder (C₆-C₁₂)-Aryl, gegebenenfalls substituiert mit (C₁-C₄)-Alkyl oder CO₂R¹¹;
- R⁵: 1. Wasserstoff oder (C₁-C₄)-Alkyl
2. 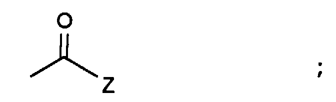
- R⁶: 1. Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, das gegebenenfalls substituiert ist mit Hydroxy, (C₁-C₄)-Alkoxy, Amino oder (C₁-C₄)-Alkylamino, oder (C₆-C₁₂)-Aryl, gegebenenfalls substituiert mit (C₁-C₄)-Alkyl oder CO₂R¹¹;
2. ein Rest der Formel (VIII): 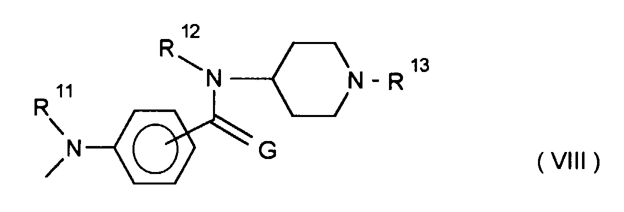
- R⁷: (C₁-C₄)-Alkyl, (C₆-C₁₂)-Aryl, oder (C₁-C₃)-Alkyl-(C₆-C₁₂)-Aryl;
- R⁸,R⁹: sind gleich oder verschieden und stehen für Wasserstoff oder Halogen;
- A: (C₁-C₃)-Alkandiyl;
- Q: O oder NR¹¹;
- R¹⁰: ein Rest der Formel (IX) 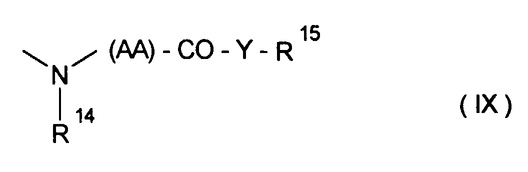
- R¹¹,R¹⁴: Wasserstoff oder (C₁-C₄)-Alkyl;
- G: O oder H_{2;}
- R¹²: steht bei G = O für Wasserstoff und bei G = H₂ für Wasserstoff oder R¹⁶CO;
- R¹³: ist (C₁-C₄)-Alkyl, (C₃-C₇)-Cycloalkyl, -(CH₂)ₘ-(C₃-C₇)-Cycloalkyl, -(CH₂)ₘ-CONR¹¹R¹¹, oder 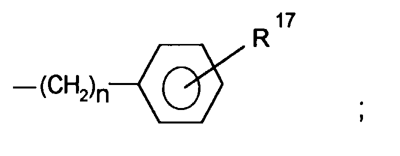
- m, n: sind gleich oder verschieden eine Zahl 0-6;
- AA: steht für eine Aminosäure wie z.B. Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, o-Methyltyrosin, β-(2-Thienyl)-alanin, Glycin, Cyclohexylalanin, Leucin, Isoleucin, Valin, Norleucin, Phenylglycin, Serin, Cystein, Aminopropionsäure oder Aminobuttersäure;
- Y: 1. (C₂-C₆)-Alkendiyl,
2. (C₁-C₈)-Alkandiyl,
3. (C₃-C₁₀)-Cycloalkendiyl,
4. -(CH)ₚ-Tₒ-(CH₂)_{q}-,
   wobei 1. bis 4. gegebenenfalls mit einen oder mehreren Resten wie z.B. O-R¹⁸, NO₂, CN, CO₂R¹¹, SO₃R¹⁸, NR²⁰R²¹, SO₂NR²⁰R²¹, CONR²⁰R²¹ substituiert sein kann;
- T: O, NR²¹, oder S;
- o: ist eine Zahl 0 oder 1;
- p,q: sind gleich oder verschieden und bedeuten eine Zahl von 0 bis 6;
- R¹⁵: 1. Wasserstoff,
2. (C₁-C₅)-Alkyl,
3. (C₆-C₁₀)-Aryl,
4. (C₁-C₉)-Heteroaryl,
   wobei 3. und 4. gegebenenfalls mit einer oder mehreren Gruppen substituiert sein können, wie Halogen, CN, NO₂, (C₁-C₆)-Alkyl, (C₆-C₁₀)-Aryl, (C₂-C₅)-Alkenyl, wobei die letzten drei Reste gegebenenfalls teilweise oder vollständig mit Halogen substituiert sein können, (C₁-C₅)-Alkoxy; (C₁-C₅)-Alkylthio, NR ²⁰R²¹, CO₂R¹⁹, SO₃R¹⁸, SO₂NR²⁰R²¹ SO₂R¹⁸, O-R¹⁸; NR²⁰CO-R¹⁵;
- R¹⁶: ist Wasserstoff, (C₁-C₄)-Alkyl, (C₆-C₁₂)-Aryl, (C₁-C₄)-Alkyl-(C₆-C₁₂)-Aryl, Perfluoro-(C₁-C₄)-Alkyl;
- R¹⁷: ist Wasserstoff, Halogen, (C₁-C₄)-Alkyl, (C₁-C₄)-Alkoxy, Perfluoro-(C₁-C₄)-Alkyl, NO₂, OH, NH₂, CONR¹⁶R¹⁶, NR¹⁶CONR¹⁶R¹⁶;
- R¹⁸,R¹⁹,R²⁰: gleich oder verschieden sind und Wasserstoff, (C₁-C₅)-Alkyl, (C₃-C₅)-Alkenyl, (C₆-C₁₂)-Aryl-(C₁-C₃)-Alkyl, (C₃-C₁₀)-Cycloalkyl, (C₃-C₁₀)-Cycloalkyl-(C₁-C₃)-Alkyl, C(O)-O-(C₁-C₅)-Alkyl, oder C(O)-NH-(C₁-C₅)-Alkyl bedeuten;
- R²¹: Wasserstoff, C(O)-O-(C₁-C₅)-Alkyl, C(O)-O-(C₁-C₃)-Alkyl-(C₆-C₁₀)-Aryl;
- Z: -R¹⁴N-R²²;
- R²²: steht für 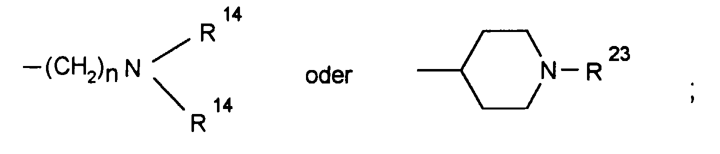
- R²³: (C₁-C₄)-Alkyl, 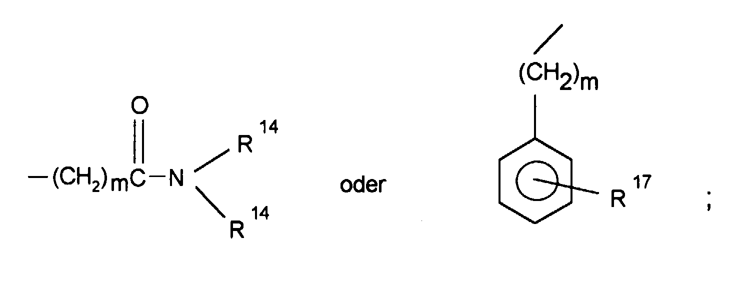
sowie deren physiologisch verträglichen Salze.

Alkyl und Alkenyl können geradkettig oder verzweigt sein. Entsprechendes gilt für davon abgeleitete Reste wie z.B. Alkoxy.

Alkenyl steht für einfach oder mehrfach ungesättigte Verbindungen wie z.B. 1,4-Butadienyl, 8,11-Heptadienyl, 8,11,14-Heptatrienyl, Butenyl.
Entsprechendes gilt für Cycloalkenyl.
Cycloalkyl steht für mono- oder bicyclische Verbindungen wie z.B. Cyclopropyl, Cyclopentyl, Cyclohexyl, Bicyclononyl. Entsprechendes gilt für Cycloalkenyl.

(C₆-C₁₂)-Aryl ist beispielsweise Phenyl, Naphthyl oder Biphenylyl, vorzugsweise Phenyl. Entsprechendes gilt auch für davon abgeleitete Reste, wie z.B. Aralkyl.

Halogen (Hal) steht für Fluor, Chlor, Brom oder Jod, vorzugsweise für Chlor oder Fluor.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Desweiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl, Benzopyranonyl, Coumarinyl, Pyranonyl, Furandionyl.

Unter physiologisch verträglichen Salzen von Verbindungen der Formel (I) versteht man sowohl deren organische als auch anorganische Salze, wie sie in Remington's Pharmaceutical Sciences (A.R. Gennard Editior, Mack Publishing Co., Easton PA, 17. Auflage, Seite 1418 (1985)) beschrieben sind. Aufgrund der physiologischen und chemischen Stabilität und der Löslichkeit sind saure Gruppen unter anderen Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Geeignete nicht-peptidische Bradykinin-Antagonisten sind zum Beispiel beschrieben in den Patentanmeldungen EP-A 622 361, US 5,212,182, US 5,216,165, US 5,438,064 und WO 9604251.

Besonders geeignet sind Verbindungen der Formel I, in welcher bedeuten:
- D: ein Rest der allgemeinen Formel (X) 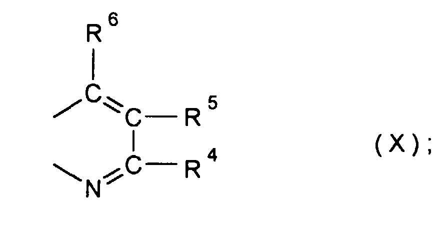
- E: 1. einen Rest der Formel (XI) 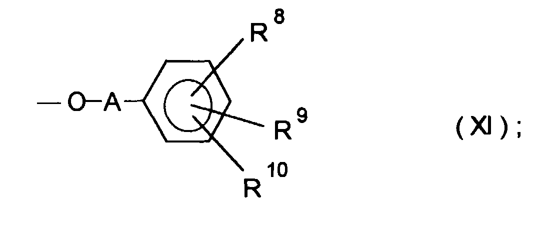
2. Wasserstoff, (C₁-C₄)-Alkyl oder (C₁-C₄)-Alkoxy;
- R¹ ,R²: sind gleich oder verschieden und stehen für Wasserstoff, Halogen oder (C₁-C₄)-Alkyl;
- R⁴: ist Wasserstoff, (C₁-C₄)-Alkyl, Phenyl oder Methoxy;
- R⁵: 1. Wasserstoff oder (C₁-C₄)-Alkyl;
2. 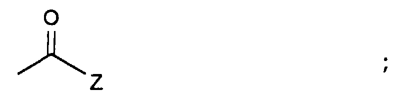
- R⁶: 1. Wasserstoff oder (C₁-C₄)-Alkyl
2. ein Rest der Formel (VIII): 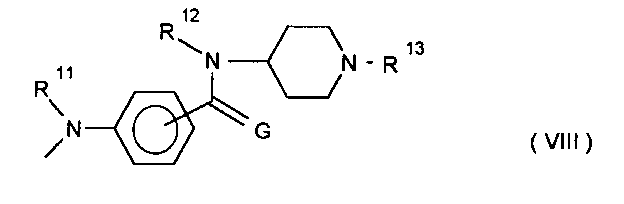
- R⁸,R⁹: sind gleich oder verschieden und stehen für Wasserstoff oder Chlor;
- A: ist -CH₂- oder -CH₂-CH₂- ;
- R¹⁰: steht für einen Rest der Formel (IX): 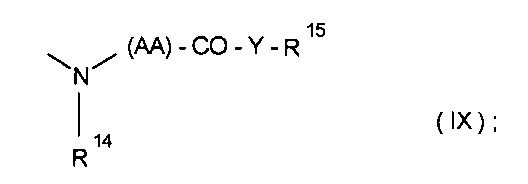
- R¹¹,R¹⁴: sind Wasserstoff, Methyl oder Ethyl;
- G: steht für O oder H₂;
- R¹²: steht bei G gleich O für Wasserstoff oder bei G gleich H₂ für Wasserstoff oder R¹⁶CO;
- R¹³: ist (C₁-C₄)-Alkyl, Cyclopentyl, Cyclohexyl, -(CH₂)ₘCONR¹¹R¹¹, 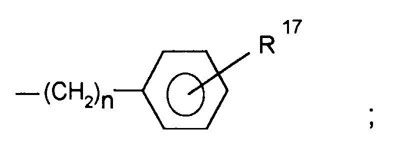
- m,n: sind gleich oder verschieden eine Zahl 0-2;
- AA: steht für die Aminosäure Glycin oder Alanin;
- Y: 1. (C₂-C₅)-Alkendiyl,
2. (C₂-C₄)-Alkandiyl,
3. -(CH₂)ₚ-Tₒ-(CH₂)_{q}- ;
- T: steht für 0 oder S;
- o: ist eine Zahl 0 oder 1;
- p,q: sind gleich oder verschieden und bedeuten eine Zahl von 0-2;
- R¹⁵: 1. Wasserstoff
2. (C₁-C₅)-Alkyl,
3. Phenyl,
4. (C₅-C₉)-Heteroaryl,
   wobei 3. und 4. gegebenenfalls mit einer, zwei oder drei Gruppen substituiert sein können, wie Halogen, NO₂, (C₁-C₃)-Alkyl, (C₁-C₃)-Alkyl, in dem die H-Atome teilweise oder vollständig durch Halogen ersetzt sind, (C₁-C₃)-Alkoxy, (C₁-C₃)-Alkylthio, NR²⁰R²¹, NR²⁰CO-(C₁-C₅)-Alkyl und NR²⁰CO-Pyridyl
- R¹⁶: ist Wasserstoff, (C₁-C₄)-Alkyl, Phenyl;
- R¹⁷: ist Wasserstoff, Halogen, (C₁-C₄)-Alkyl, NO₂, NH₂;
- R²⁰: ist Wasserstoff, (C₁-C₄)-Alkyl, Benzyl;
- R²¹: ist Wasserstoff, C(O)-O-(C₁-C₅)-Alkyl;
- Z: ist -R¹⁴-N-R²²
- R²²: 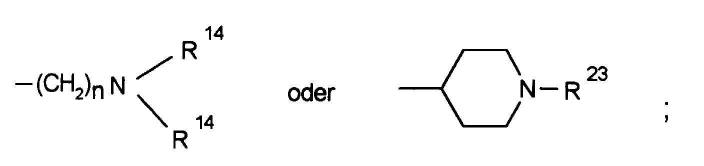
- R²³: (C₁-C₄)-Alkyl, 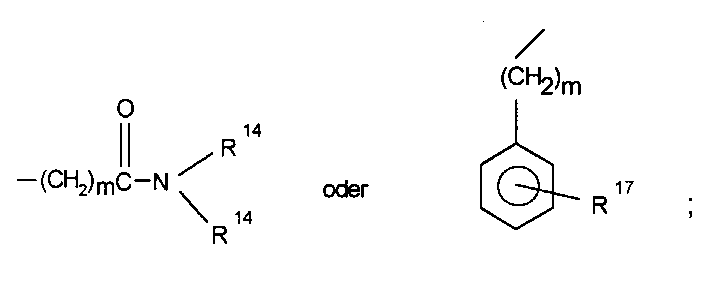
sowie deren physiologisch verträglichen Salze.

Besonders geeeignet sind auch Verbindungen der Formel (I), in welcher die Symbole die folgende Bedeutung haben:
- D: ein Rest der allgemeinen Formel (X) 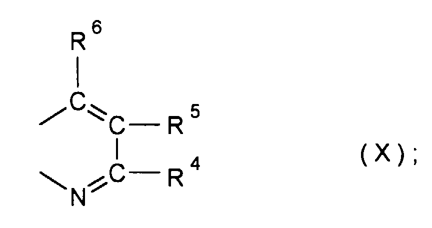
- E: ein Rest der Formel (XI) 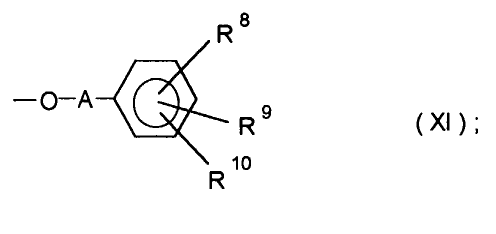
- R¹,R²: sind gleich oder verschieden und stehen für Wasserstoff, Halogen oder (C₁-C₄)-Alkyl;
- R⁴: Wasserstoff, (C₁-C₄)-Alkyl;
- R⁵: Wasserstoff;
- R⁶: Wasserstoff;
- R⁸,R⁹: sind gleich oder verschieden und stehen für Wasserstoff oder Chlor;
- A: -CH₂- oder -CH₂-CH₂- ;
- R¹⁰: ein Rest der Formel (IX): 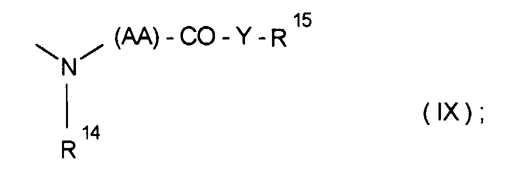
- R¹⁴: Wasserstoff, Methyl oder Ethyl;
- AA: steht für die Aminosäure Glycin;
- Y: 1. (C₂-C₅)-Alkendiyl,
2. (C₂-C₄)-Alkandiyl,
3. -(CH₂)ₚ-Tₒ-(CH₂)_{q}- ;
- T: steht für 0 oder S;
- o: ist eine Zahl 0 oder 1;
- p,q: sind gleich oder verschieden und bedeuten eine Zahl von 0-2;
- R¹⁵: 1. Wasserstoff
2. (C₁-C₃)-Alkyl,
3. Phenyl,
4. (C₅-C₉)-Heteroaryl,
   wobei 3. und 4. gegebenenfalls mit einer, zwei oder drei Gruppen substituiert sein können, wie Halogen, NO₂, (C₁-C₃)-Alkyl, (C₁-C₃)-Alkyl, in dem die H-Atome teilweise oder vollständig durch Halogen ersetzt sind, (C₁-C₃)-Alkoxy, NR²⁰R²¹, NR²⁰CO- (C₁-C₃)-Alkyl und NR²⁰CO-Pyridyl;
- R²⁰: ist Wasserstoff, (C₁-C₄)-Alkyl, Benzyl;
- R²¹: ist Wasserstoff, C(O)-O-(C₁-C₅)-Alkyl;
sowie deren physiologisch verträglichen Salze.

Geeignet sind auch die Verbindungen
N-[1-[4-(1,1-Dimethylethyl)phenyl]methyl-4-piperidinyl]-8-methoxy-4-[[4-[[[1-(phenylmethyl)-4-piperidinyl]amino]carbonyl]phenyl-amino]-3- chinolincarboxylamid;
N-[1-[(3-chlorphenyl]methyl-4-piperidinyl]-8-methoxy-4-[[4-[[[1-(phenylmethyl)-4-piperidinyl]amino]carbonyl]phenyl-amino]-3-chinolincarboxylamid;
8-Methoxy-N-[1-(phenyl]methyl-4-piperidinyl-[4-[[4-[[[1-(phenylmethyl)-4-piperidinyl]amino]carbonyl]phenyl-amino]-3-chinolincarboxylamid;
N-[2-(Dimethylamino)ethyl]-8-methoxy-4-[[4-[[[1-(phenylmethyl)-4-piperidinyl]amino]carbonyl]phenyl-amino]-3-chinolincarboxylamid-trifluoracetat;
N-[2-(Dimethylamino)ethyl]-N-ethyl-8-methoxy-4-[[4-[[[1-(phenylmethyl)-4-piperidinyl]amino]carbonyl]phenyl-amino]-3-chinolincarboxylamid;
4-[[4-[[[(3-Cyclopentyl-1-oxopropyl)-[1-[6-(diethylamino)-6-oxo-hexyl-4-piperidinylamino]-methylphenyl]-amino]-8-methoxy-N-[1-(phenylmethyl)-4-piperidinyl]-3-chinolincarboxylamid;
4-[[4-[[(1-Butyl-4-piperidinylamino]methyl]-phenylamino]-8-methoxy-N-[1-(phenylmethyl)-4-piperidinyl]-3-chinolincarboxylamid;
N-(1-Butyl-4-piperidinyl)-8-methoxy-[[4-[[[1-(phenylmethyl)-4-piperidinyl]amino]-carbonyl]-phenyl]-amino]-3-chinolincarboxylamid;
N-[1-[6-(Diethylamino)-6-oxohexyl-4-piperidinyl-8-methoxy-4-[[4-[[[1-(phenylmethyl)-4-piperidinyl]-amino]-carbonyl]-phenyl]-amino]-3-chinolincarboxylamid;
4-[[4-[[(1-Butyl-4-piperidinyl)-(1-oxobutyl)-amino]-methyl]-phenyl]-amino-[8-methoxy-N-[1-(phenylmethyl)-4-piperidinyl]-3-chinolincarboxylamid;
N-[1-[4-[(Diethylamino)-carbonyl]-phenyl-4-piperidinyl-8-methoxy-4-[[4[[[1-(phenylmethyl)-4-piperidinyl]-amino]-carbonyl]-phenyl]-amino-3-chinolincarboxylamid;
N-[1-(2-Phenylethyl)-4-piperidinyl]-8-methoxy-4-[[4-[[[1-(phenylmethyl)-4-piperidinyl]-amino]-carbonyl]-phenyl]-amino]-3-chinolincarboxylamid;
4-[[4-[[(1-Butyl-4-piperidinyl)-amino]-carbonyl]-phenyl]-amino]-8-methoxy-N-[1-(phenylmethyl)-4-piperidinyl]-3-chinolincarboxylamid;
8-Methoxy-N-(1-methyl-4-piperidinyl)-4-[[4-[[[1-(phenylmethyl)-4-piperidinyl]-amino]-carbonyl]-phenyl]-amino]-3-chinolincarboxylamid;
N-[1-[(3-Methoxyphenyl)-methyl]-4-piperidinyl-8-methoxy-4-[[4-[[[1-(phenylmethyl)-4-piperidinyl]-amino]-carbonyl]-phenyl]-amino-3-chinolincarboxylamid;
8-Methoxy-4-[[4-[[[1-(phenylmethyl)-4-piperidinyl]-amino]-carbonyl]-phenyl]-amino]-1-[[3-(trifluoromethyl)-phenyl]-methyl]-4-piperidinyl]-3-chinolincarboxylamid; oder
7-Chloro-N-[1-(phenylmethyl)-4-piperidinyl]-4-[[4-[[[1-(phenylmethyl)-4-piperidinyl]-amino]-carbonyl]-phenyl]-amino]-3-chinolincarboxamid;
sowie deren physiologisch verträglichen Salze.

Ganz besonders geeignet sind auch die folgenden, in den Tabellen 1-3 aufgelisteten Verbindungen der Beispiele 1-45.

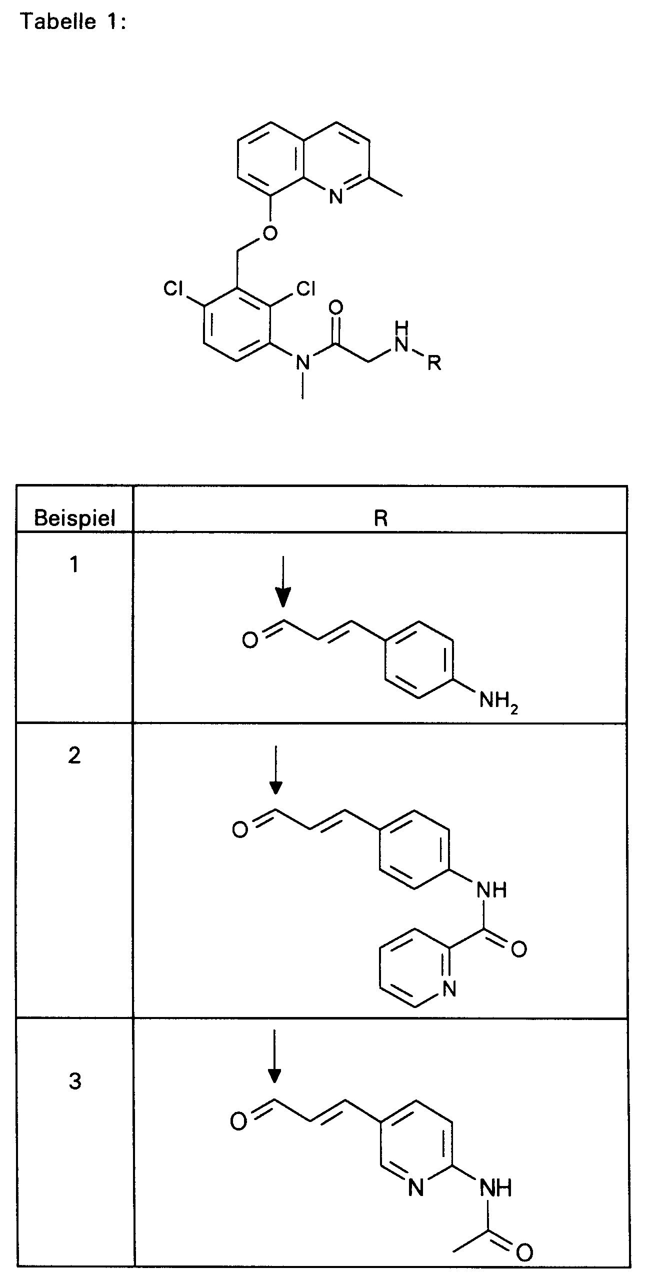

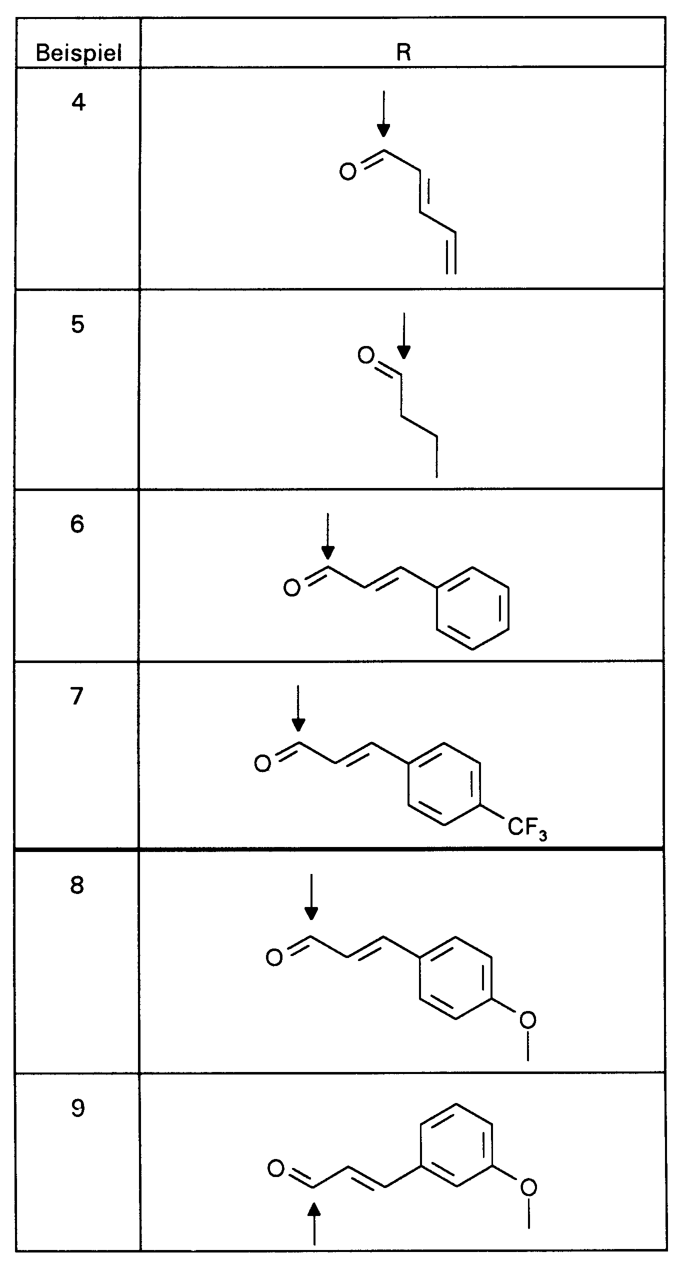

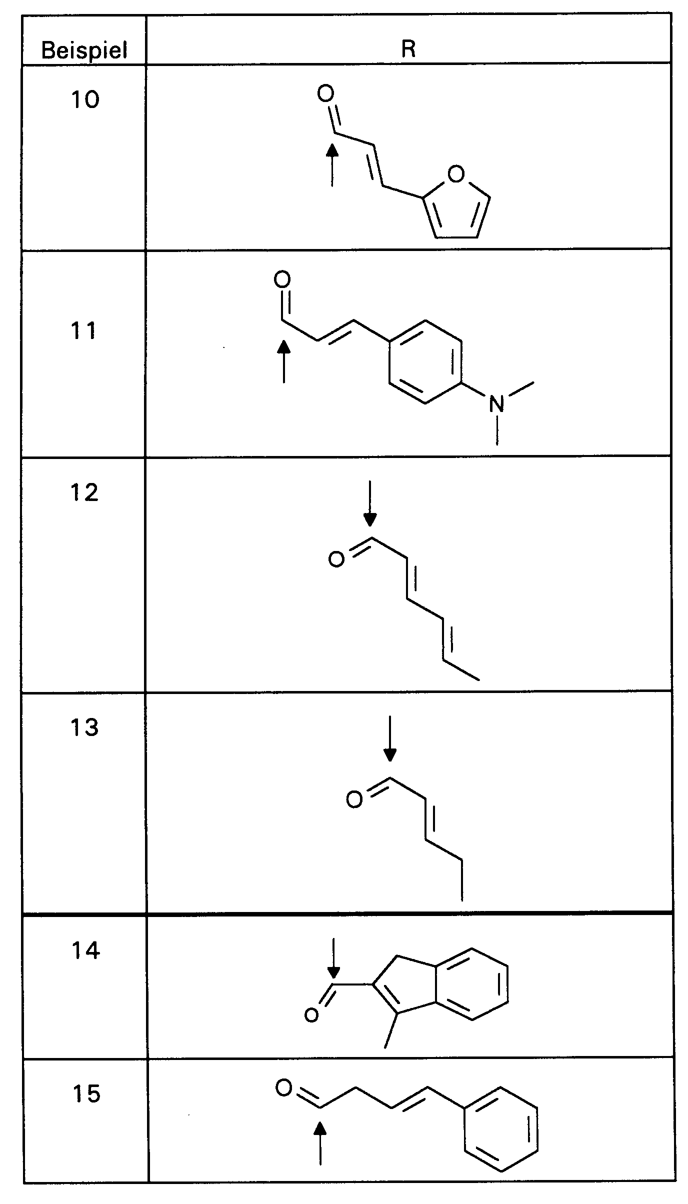

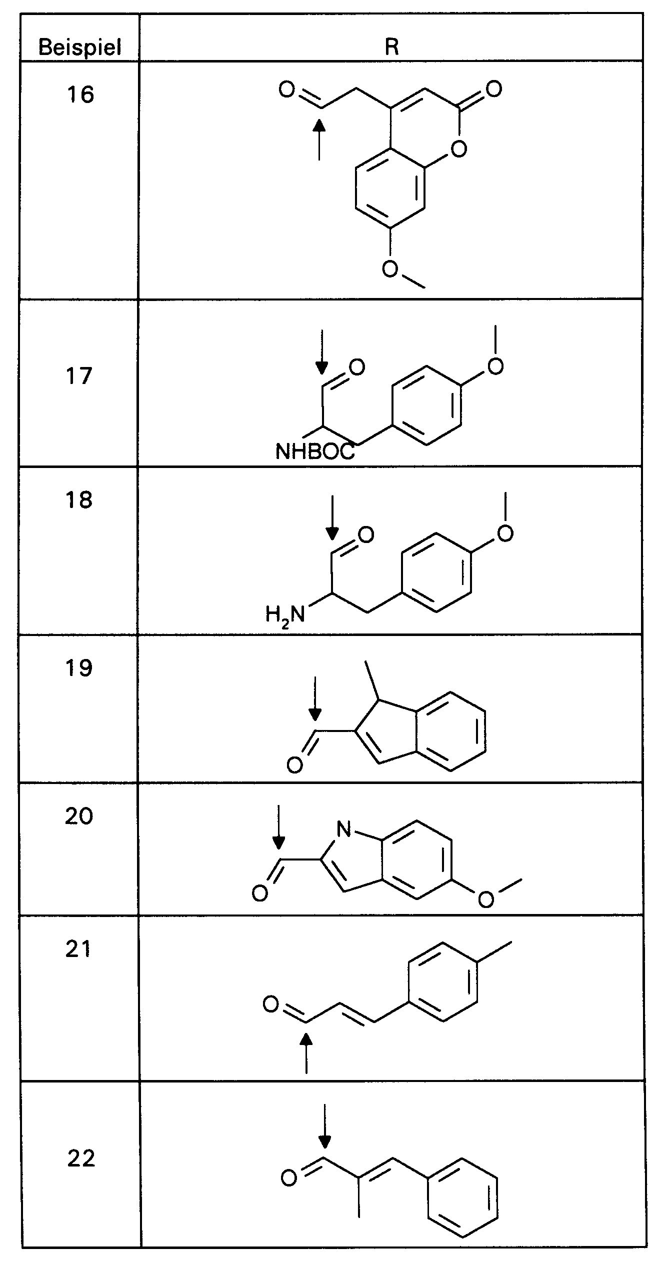

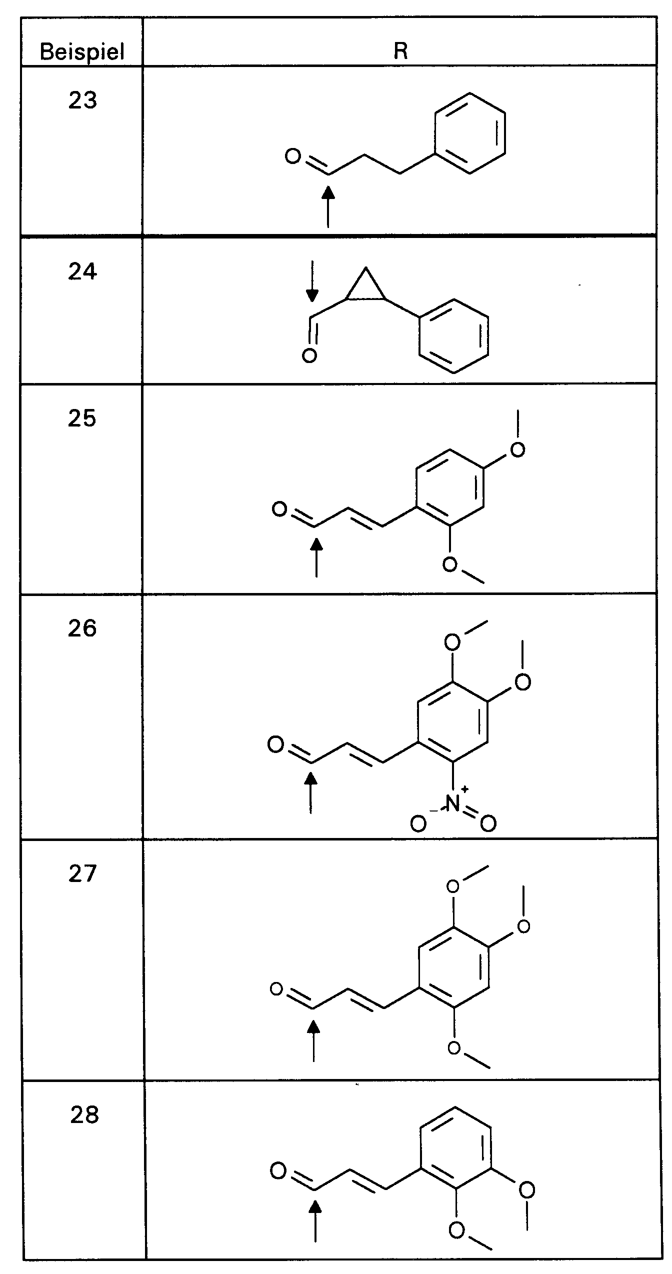

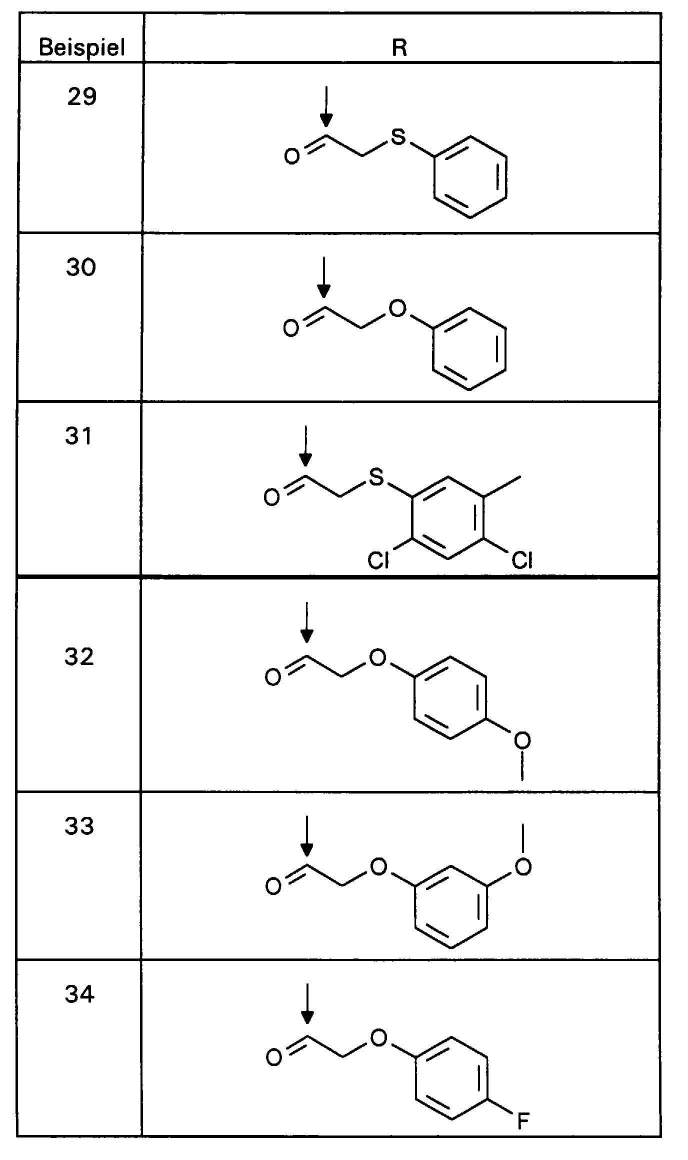

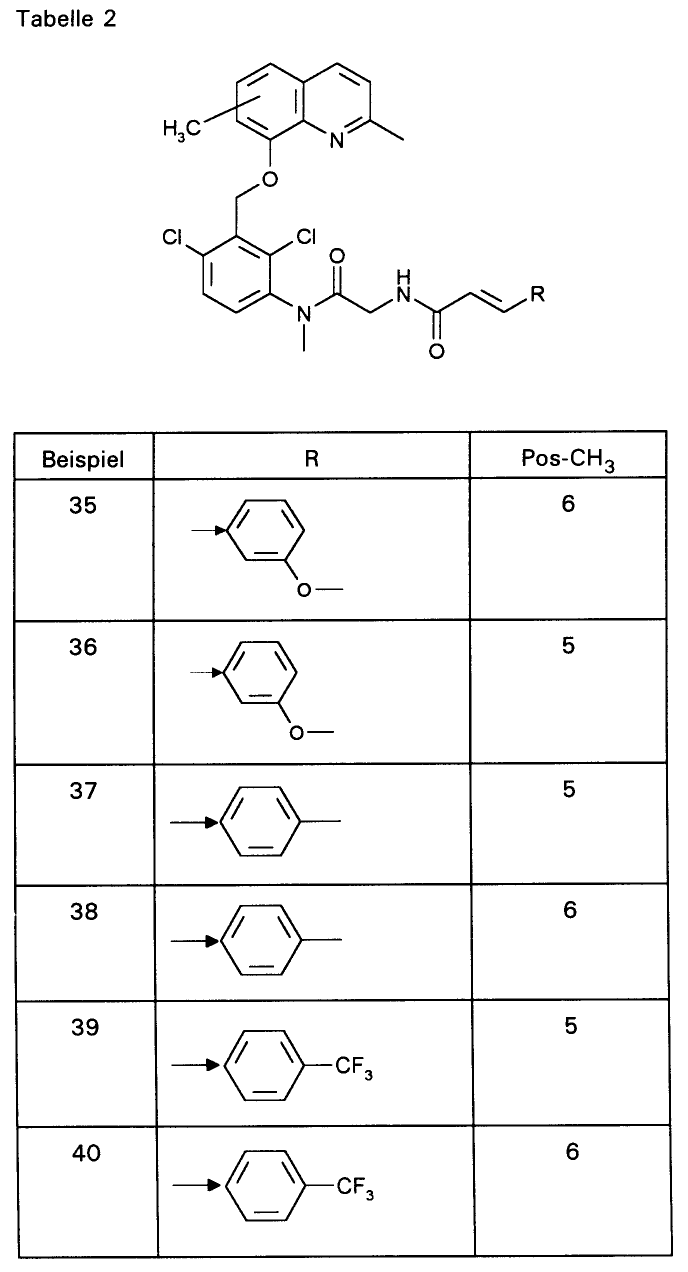

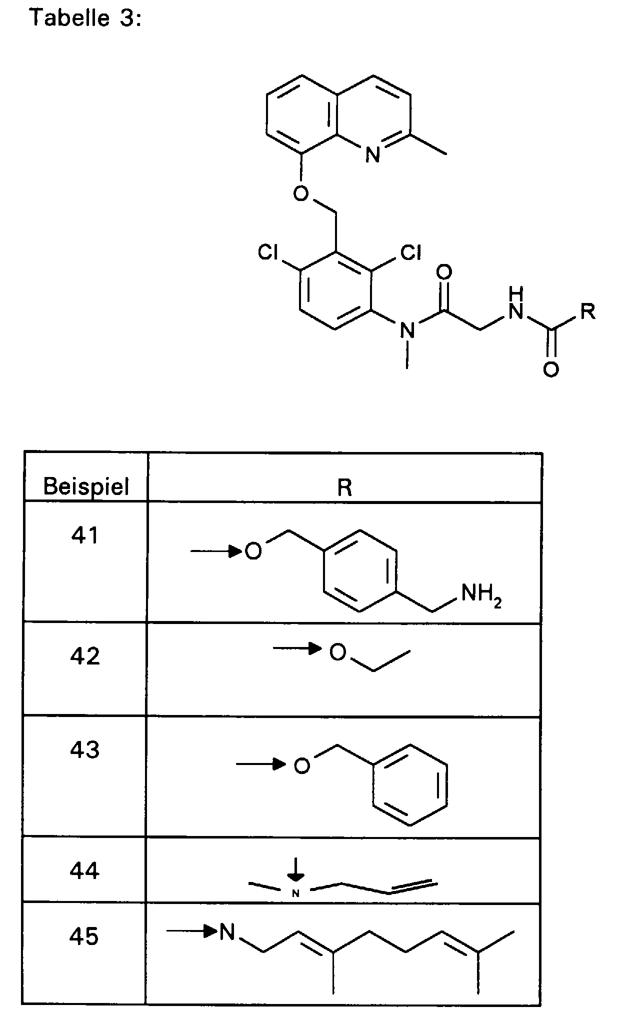

Die Anwendung kann enteral, parenteral - wie z.B. subkutan, im. oder i.v. -, nasal, rektal oder per Inhalation erfolgen. Die Dosierung des Wirkstoffs hängt vom Körpergewicht, Alter und von der Applikationsart ab.

Die pharmazeutischen Präparate der vorliegenden Erfindung werden in an sich bekannten Lösungs-, Misch-, Granulier-, Tabletten- oder Dragierverfahren hergestellt.

Zur parenteralen Applikation werden die aktiven Verbindungen oder deren physiologisch verträgliche Salze, gewünschtenfalls mit den pharmazeutisch üblichen Hilfsstoffen, beispielsweise zur Isotonisierung oder pH-Einstellung sowie Lösungsvermittler, Emulgatoren, oder anderen Hilfsstoffen, in Lösung, Suspension oder Emulsion gebracht.

Für die beschriebenen Arzneistoffe ist auch der Einsatz von injizierbaren Retardzubereitungen zur subkutanen oder intramuskulären Anwendung sinnvoll. Als Arzneimittel können z.B. ölige Kristallsuspensionen, Mikrokapseln, Mikropartikel, Nanopartikel oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren aufgebaut sein können. Andere denkbare Polymere sind Polyamide, Polyester, Polyacetate oder Polysaccharide.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung von festen Arzneiformen sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Auch orale Retardzubereitungen oder Zubereitungen mit magensaftresistenten Überzügen sind denkbar. Retardzubereitungen können auf Basis von Fett-, Wachs- oder Polymereinbettungen aufgebaut sein. Möglich sind hierbei auch Mehrschicht- oder Manteltabletten oder Pellets.

Für die beschriebenen Arzneistoffe ist auch eine Applikation auf Schleimhäute zur Erzielung systemisch wirksamer Spiegel sinnvoll. Dies betrifft die Möglichkeit der intranasalen, inhalativen und rektalen Anwendung.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie Pulver, wäßrige, alkoholische oder ölige Suspensionen oder wäßrige, alkoholische oder ölige Lösungen. Wäßrigen intranasalen Zubereitungen können Chelatbildner, wie Ethylendiamin-N,N,N',N'-tetraessigsäure und Puffer wie Essigsäure, Phosphorsäure, Citronensäure, Weinsäure und deren Salze zugefügt werden. Mehrfachdosenbehälter enthalten Konservierungsmittel wie Benzalkoniumchlorid, Chlorbutanol, Chlorhexidin, Sorbinsäure, Benzoesäure, PHB-Ester oder Organoquecksilberverbindungen.

Die Applikation der Nasallösungen kann mittels Dosierzerstäuber erfolgen oder als Nasaltropfen mit viskositätserhöhendem Anteil bzw. Nasengels oder Nasencremes. Für die inhalative Anwendung können Vernebler oder Druckgas-Packungen unter Verwendung inerter Trägergase benutzt werden.

Zur Applikation von Pulvern zur nasalen oder pulmonalen Inhalation sind spezielle Applikatoren erforderlich.

Die wirksame Dosis der Verbindungen der Formel (I) beträgt mindestens 0,01 mg/kg/Tag, vorzugsweise mindestens 0,1 mg/kg/Tag, höchstens 30 mg/kg/Tag, vorzugsweise 0,3 bis 10 mg/kg/Tag Körpergewicht in Abhängigkeit vom Schweregrad der Symptomatik, bezogen auf einen Erwachsenen von 75 kg Körpergewicht.

### Versuch:

Wirkung der Verbindung aus Beispiel 7 (= Verbindung A) auf die Urin- und Elektrolytausscheidung an Ratten mit Tetrachlorkohlenstoff-induzierter Leberfibrose.
1) Methode
   An Wistar Ratten (Züchter: Hoechst AG, Kastengrund) mit einem anfänglichen Körpergewicht von 120-150 g wurde, wie von Bickel et al. (J. Hepatol. 1991; 13 (Suppl. 3), S26-S33) beschrieben, eine Leberfibrose induziert. Dazu erhielten die Tiere zweimal pro Woche Tetrachlorkohlenstoff (CCl₄) in einer Dosis von 1 ml/kg per os für mindestens 6 Wochen. Die Fibrose der Leber wurde über den Kollagengehalt der Leber und Leber-relevante Serumparameter (Bilirubin, ALAT, Gallensäuren) verifiziert.
   Im Verlauf der Fibrogenese wurden die Tiere unter Standardbedingungen wie folgt gehalten: Tages-Nacht-Rhythmus (Hellphase von 6.30 bis 18.30), Raumtemperatur 22 ± 2°C und relative Luftfeuchtigkeit 60 ± 10 %. Die Tiere erhielten standartisierte Rattenfutter (Altromin® 1321) und Wasser ad libitum.
2) Salurese- und Diureseversuch:
   Zum Zeitpunkt des Diureseversuches hatten die Tiere ein Gewicht zwischen 210 und 260 g erreicht. Futter wurde bereits 16 h vor dem Versuch entzogen und während des gesamten Versuchs vorenthalten. Der freie Zugang zu Wasser war den Tieren noch bis zum eigentlichen Versuchsbeginn gestattet.
   Die Tiere wurden für die Dauer des Diureseversuches in speziellen Diuresekäfigen gehalten. Eine kontrollierte Diurese wurde mit einer oralen Gabe von 20 ml Wasser per kg Körpergewicht zum Zeitpunkt 0 h ausgelöst. Die Ausscheidung von Elektrolyten und das Urinvolumen wurden in den Sammelperioden von 0-5 und 6-24 h getrennt für jedes Tier ermittelt.
   Sieben Tage später wurde der Versuch an den gleichen Tieren erneut durchgeführt unter Gabe von Bradykininantagonisten. Zum Zeitpunkt 0 und 6 h erhielten die Tiere 3 mg/kg Körpergewicht Verbindung A intraperitoneal, gelöst in 5 ml/kg Körpergewicht mit der folgenden Zusammensetzung: DSMO 28%, Ethanol 20%, Aqua bidest 44% und 0,9%ige Kochsalzlösung 8%, pH 5,71.
   Natrium und Kalium wurden flammenphotometrisch bestimmt (Flammenphotometer Eppendorf, Hamburg). Chlorid wurde argentometrisch über potentiometrische Endpunktbestimmung gemessen (Chloridmeter Eppendorf, Hamburg). Die analytischen Ergebnisse wurden zur Berechnung der Urinausscheidung (ml/kg Körpergewicht) und Elektrolytausscheidung (mmol/kg Körpergewicht) herangezogen.
3) Ergebnis:
   Wirkung von Verbindung A auf die Urin- und Elektrolytausscheidung an Ratten mit Tetrachlorkohlenstoff-induzierter Leberfibrose 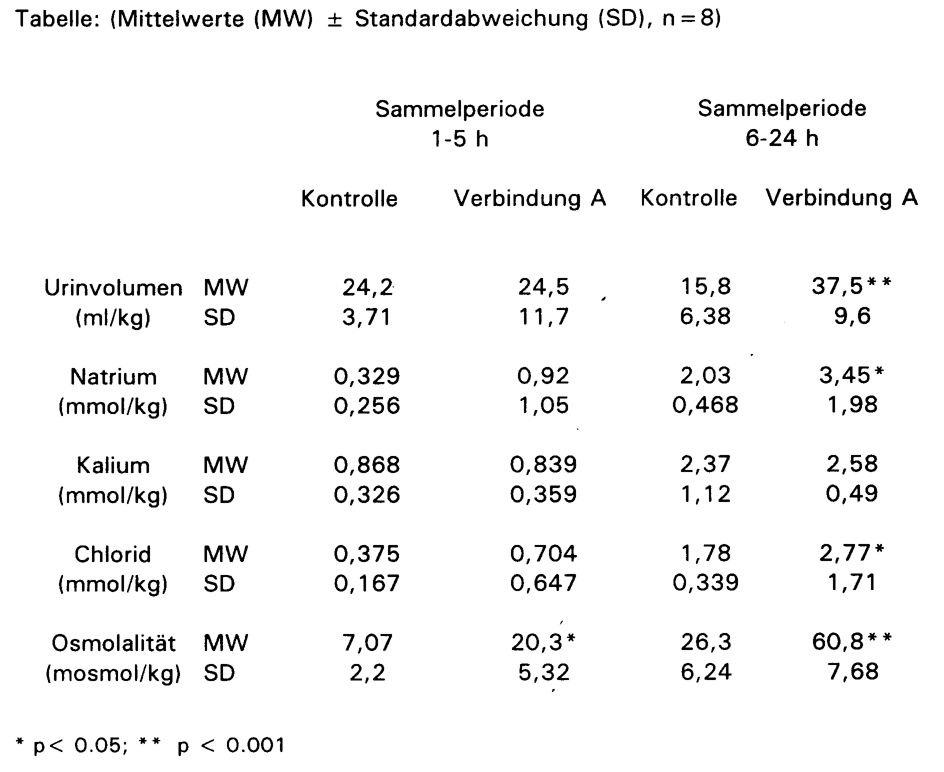
4) Statistik:
   Die Ergebnisse sind angegeben als arithmetische Mittel und Standardabweichung (SD). Die statistische Überprüfung erfolgte mit dem nicht-parametrischen Test nach Mann-Whitney.
5) Bewertung:
   Ratten mit Leberfibrose zeigen eine deutliche Erhöhung der Diurese und Salurese nach Behandlung mit nicht-peptidischen Bradykininantagonisten. Als Beispiel sind in der obigen Tabelle experimente Daten mit der Beispielverbindung 6 (Verbindung A) aufgeführt. Es ergibt sich eine deutliche, statistisch signifikante Zunahme der Diurese und Ausscheidung von Natrium und Chlorid.

Das Modell der Tetrachlorkohlenstoff-induzierten Leberfibrose an der Ratte findet als Modell für die Leberzirrhose am Menschen allgemeine Anerkennung. Überschießende Natriumretention ist charakteristisch für die Leberfibrose und Leberzirrhose bei Mensch und Tier und wird als Folge einer tiefgreifenden hämodynamischen Störung betrachtet (Schrier et al., Hepatology 1988; 1151-1157). Diese hämodynamische Störung besteht in einer portalen Hypertonie (Pfortaderhochdruck), eng verknüpft mit einer überschießenden peripheren Vasodilation vor allem im Splanchnicusgebiet (hyperdyname Kreislaufsituation). Die Ursache der peripheren Vasodilatation war bisher nicht geklärt. Die pathologische Natrium- und Wasserretention verschlechtert ihrerseits die Symptomatik, indem sie z.B. zu Ödembildung und Aszites beiträgt. Die portale Hypertonie ist assoziiert mit inadäquater peripherer Vasodilatation und Natriumretention. Diese werden für Dekompensationserscheinungen bei Leberfibrose und Leberzirrhose verantwortlich gemacht. Diese Dekompensationserscheinungen umfassen nicht nur Symptome wie Ödembildung und Aszites, sondern auch das sogenannte hepatorenale Syndrom (Nierenversagen als Folge einer schweren Lebererkrankung).

Die starke natriuretische Wirkung von nicht-peptidischen Bradykininantagonisten der Formel (I) bei Ratten mit Leberfibrose und Leberzirrhose ist unerwartet, weil Bradykininantagonisten an gesunden Tieren diese Wirkung nicht zeigen und, im Gegenteil, in besonderen Hypertonie-Modellen sogar zu einer Verminderung der Diurese und Natriumausscheidung führen kann (Madeddu et al., Br. J. Pharmacol. 1992; 106: 380-86; Majima et al., Hypertension 1993; 22, 705-714). Bradykinin kann nämlich in der Niere Salurese und Diurese über vaskuläre und tubuläre Mechanismen stimulieren.

Bradykinin ist ein endogenes Peptid mit stark vasodilatierenden und gefäßpermeabilitätssteigernden Eigenschaften in verschiedenen Gefäßgebieten. Unsere Ergebnisse zeigen, daß Bradykinin ein wesentlicher Mediator der exzessiven Natriumretention ist. Eine verbesserte hämodynamische und mikrovaskuläre Situation überkompensiert bei weitem eine mögliche Einschränkung der Natrium- und Wasserausscheidung durch Hemmung der stimulierenden Wirkung endogenen Bradykinins in der Niere, so daß ein therapeutischer Nutzen resultiert.

Somit sind nicht-peptidische Bradykininantagonisten der Formel (I) geeignet für die therapeutische und präventive Behandlung bei chronischen fibrogenetischen Lebererkrankungen (Leberzirrhose und Leberfibrose) und akuten Lebererkrankungen.

## Patentansprüche

1. Verwendung von nicht-peptidischen Bradykininantagonisten oder deren physiologisch verträglichen Salze zur Herstellung von Arzneimitteln zur Behandlung von chronisch-fibrogenetischen Lebererkrankungen (Leberzirrhose und Leberfibrose) und akuten Lebererkrankungen und zur Prävention von Komplikationen.

2. Verwendung nach Anspruch 1 eines nicht-peptidischen Bradykininantagonisten der Formel (I) 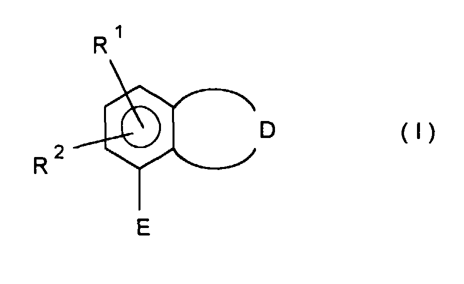 in welcher die Symbole folgende Bedeutung haben:
D
1. ein Rest der Formel (II): 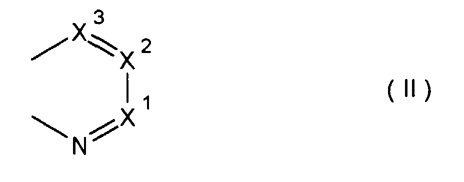
2. ein Rest der Formeln (III) bis (VI): 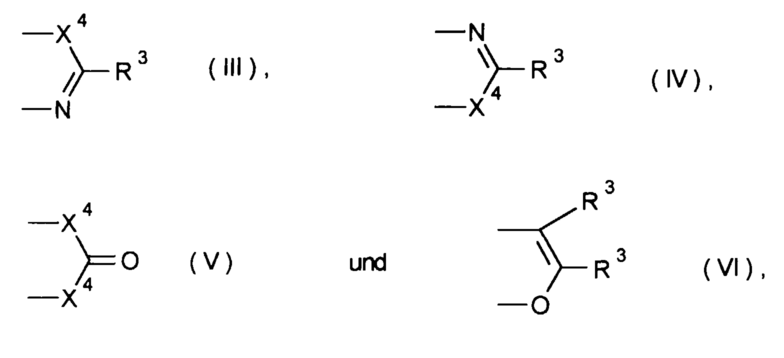
E
1. ein Rest der Formel (VII): 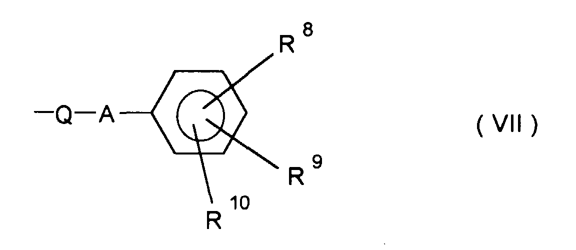
2. Wasserstoff, Halogen, (C₁-C₄)-Alkyl, (C₁-C₄)-Alkylthio, (C₁-C₄)-Alkoxy, wobei in den letzten 3 Resten 1 oder mehrere Wasserstoffatome durch Fluor ersetzt sein können;
X¹ Stickstoff oder C-R⁴ ;
X² Stickstoff oder C-R⁵ ;
X³ Stickstoff oder C-R⁶ ;
X⁴ Sauerstoff, Stickstoff oder N-R⁷ ;
R¹,R² sind gleich oder verschieden und stehen für Wasserstoff, Halogen, (C₁-C₄)-Alkyl oder (C₁-C₄)-Alkoxy;
R³ Wasserstoff, Halogen, (C₁-C₆)-Alkyl, (C₆-C₁₂)-Aryl, (C₁-C₃)-Alkyl-(C₆-C₁₂)-Aryl, (C₃-C₅)-Alkenyl, (C₁-C₄)-Alkoxy, CO₂R¹¹;
R⁴ Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, das gegebenenfalls substituiert ist mit Hydroxy, (C₁-C₄)-Alkoxy, Amino oder (C₁-C₄)-Alkylamino, oder (C₆-C₁₂)-Aryl, gegebenenfalls substituiert mit (C₁-C₄)-Alkyl oder CO₂R¹¹;
R⁵
1. Wasserstoff oder (C₁-C₄)-Alkyl
2. 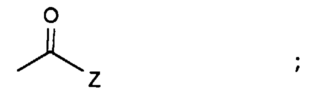
R⁶
1. Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, das gegebenenfalls substituiert ist mit Hydroxy, (C₁-C₄)-Alkoxy, Amino oder (C₁-C₄)-Alkylamino, oder (C₆-C₁₂)-Aryl, gegebenenfalls substituiert mit (C₁-C₄)-Alkyl oder CO₂R¹¹;
2. ein Rest der Formel (VIII): 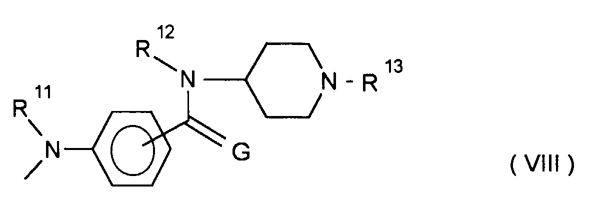
R⁷ (C₁-C₄)-Alkyl, (C₆-C₁₂)-Aryl, oder (C₁-C₃)-Alkyl-(C₆-C₁₂)-Aryl;
R⁸,R⁹ sind gleich oder verschieden und stehen für Wasserstoff oder Halogen;
A (C₁-C₃)-Alkandiyl;
Q O oder NR¹¹;
R¹⁰ ein Rest der Formel (IX) 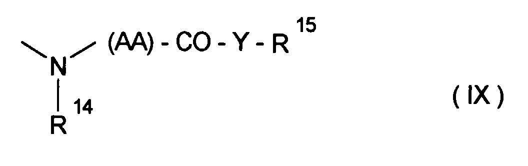
R¹¹,R¹⁴ Wasserstoff oder (C₁-C₄)-Alkyl;
G O oder H₂;
R¹² steht bei G = O für Wasserstoff und bei G = H₂ für Wasserstoff oder R¹⁶CO;
R¹³ ist (C₁-C₄)-Alkyl, (C₃-C₇)-Cycloalkyl, -(CH₂)ₘ-(C₃-C₇)-Cycloalkyl, -(CH₂)ₘ-CONR¹¹R¹¹, oder 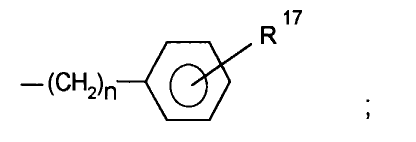
m, n sind gleich oder verschieden eine Zahl 0-6;
AA steht für eine Aminosäure wie z.B. Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, o-Methyltyrosin, β-(2-Thienyl)-alanin, Glycin, Cyclohexylalanin, Leucin, Isoleucin, Valin, Norleucin, Phenylglycin, Serin, Cystein, Aminopropion-säure oder Aminobuttersäure;
Y
1. (C₂-C₆)-Alkendiyl,
2. (C₁-C₈)-Alkandiyl,
3. (C₃-C₁₀)-Cycloalkendiyl,
4. -(CH)ₚ-Tₒ-(CH₂)_{q}-,
wobei 1. bis 4. gegebenenfalls mit einen oder mehreren Resten wie z.B. O-R¹⁸, NO₂, CN, CO₂R¹¹, SO₃R¹⁸, NR²⁰R²¹, SO₂NR²⁰R²¹, CONR²⁰R²¹ substituiert sein kann;
T O, NR²¹, oder S;
o ist eine Zahl 0 oder 1
p,q sind gleich oder verschieden und bedeuten eine Zahl von 0 bis 6;
R¹⁵
1. Wasserstoff,
2. (C₁-C₅)-Alkyl,
3. (C₆-C₁₀)-Aryl,
4. (C₁-C₉)-Heteroaryl,
wobei 3. und 4. gegebenenfalls mit einer oder mehreren Gruppen substituiert sein können, wie Halogen, CN, NO₂, (C₁-C₆)-Alkyl, (C₆-C₁₀)-Aryl, (C₂-C₅)-Alkenyl, wobei die letzten drei Reste gegebenenfalls teilweise oder vollständig mit Halogen substituiert sein können, (C₁-C₅)-Alkoxy; (C₁-C₅)-Alkylthio, NR ²⁰R²¹, CO₂R¹⁹, SO₃R¹⁸, SO₂NR²⁰R²¹, SO₂R¹⁸, O-R¹⁸; NR²⁰CO-R¹⁵;
R¹⁶ ist Wasserstoff, (C₁-C₄)-Alkyl, (C₆-C₁₂)-Aryl, (C₁-C₄)-Alkyl-(C₆-C₁₂)-Aryl, Perfluoro-(C₁-C₄)-Alkyl;
R¹⁷ ist Wasserstoff, Halogen, (C₁-C₄)-Alkyl, (C₁-C₄)-Alkoxy, Perfluoro-(C₁-C₄)-Alkyl, NO₂, OH, NH₂, CONR¹⁶R¹⁶, NR¹⁶CONR¹⁶R¹⁶;
R¹⁸,R¹⁹,R²⁰ gleich oder verschieden sind und Wasserstoff, (C₁-C₅)-Alkyl, (C₃-C₅)-Alkenyl, (C₆-C₁₂)-Aryl-(C₁-C₃)-Alkyl, (C₃-C₁₀)-Cycloalkyl, (C₃-C₁₀)-Cycloalkyl-(C₁-C₃)-Alkyl, C(O)-O-(C₁-C₅)-Alkyl, oder C(O)-NH-(C₁-C₅)-Alkyl bedeuten;
R²¹ Wasserstoff, C(O)-O-(C₁-C₅)-Alkyl, C(O)-O-(C₁-C₃)-Alkyl-(C₆-C₁₀)-Aryl;
Z -R¹⁴N-R²²;
R²² steht für 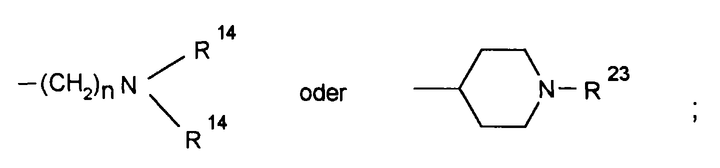
R²³ (C₁-C₄)-Alkyl, 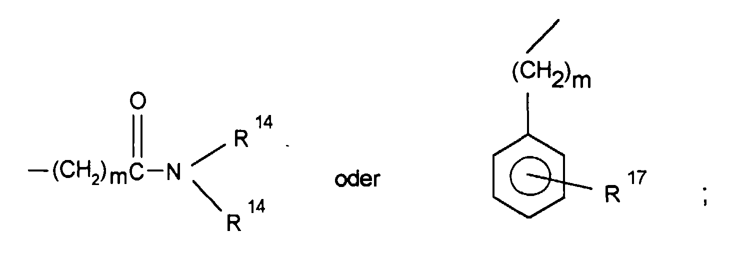
sowie deren physiologisch verträglichen Salze.

3. Verwendung nach Anspruch 2 eines nicht-peptidischen Bradykininantagonisten der Formel (I), in welcher die Symbole folgende Bedeutung haben:
D ein Rest der allgemeinen Formel (X) 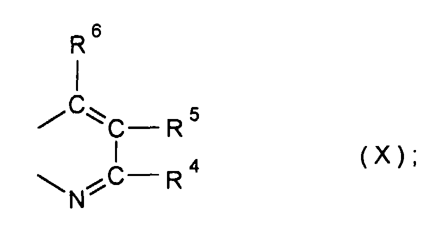
E
1. einen Rest der Formel (XI) 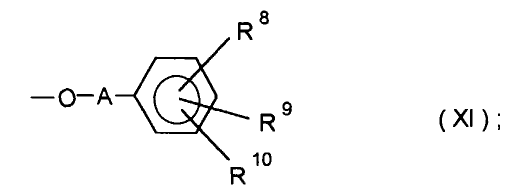
2. Wasserstoff, (C₁-C₄)-Alkyl oder (C₁-C₄)-Alkoxy;
R¹,R² sind gleich oder verschieden und stehen für Wasserstoff, Halogen oder (C₁-C₄)-Alkyl;
R⁴ ist Wasserstoff, (C₁-C₄)-Alkyl, Phenyl oder Methoxy;
R⁵
1. Wasserstoff oder (C₁-C₄)-Alkyl;
2. 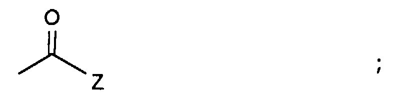
R⁶
1. Wasserstoff oder (C₁-C₄)-Alkyl
2. ein Rest der Formel (VIII): 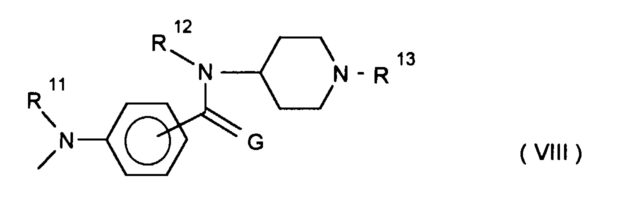
R⁸,R⁹ sind gleich oder verschieden und stehen für Wasserstoff oder Chlor;
A ist -CH₂- oder -CH₂-CH₂- ;
R¹⁰ steht für einen Rest der Formel (IX): 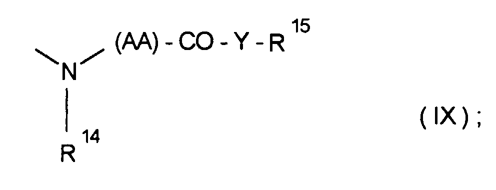
R¹¹,R¹⁴ sind Wasserstoff, Methyl oder Ethyl;
G steht für O oder H₂;
R¹² steht bei G gleich O für Wasserstoff oder bei G gleich H₂ für Wasserstoff oder R¹⁶CO;
R¹³ ist (C₁-C₄)-Alkyl, Cyclopentyl, Cyclohexyl, -(CH₂)ₘCONR¹¹R¹¹, 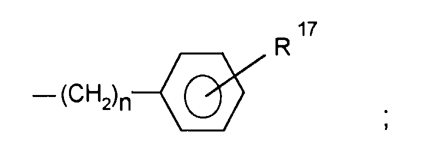
m,n sind gleich oder verschieden eine Zahl 0-2;
AA steht für die Aminosäure Glycin oder Alanin;
Y
1. (C₂-C₅)-Alkendiyl,
2. (C₂-C₄)-Alkandiyl,
3. -(CH₂)ₚ-Tₒ-(CH₂)_{q}- ;
T steht für 0 oder S;
o ist eine Zahl 0 oder 1;
p,q sind gleich oder verschieden und bedeuten eine Zahl von 0-2;
R¹⁵
1. Wasserstoff
2. (C₁-C₅)-Alkyl,
3. Phenyl,
4. (C₅-C₉)-Heteroaryl,
wobei 3. und 4. gegebenenfalls mit einer, zwei oder drei Gruppen substituiert sein können, wie Halogen, NO₂, (C₁-C₃)-Alkyl, (C₁-C₃)-Alkyl, in dem die H-Atome teilweise oder vollständig durch Halogen ersetzt sind, (C₁-C₃)-Alkoxy, (C₁-C₃)-Alkylthio, NR²⁰R²¹, NR²⁰CO-(C₁-C₅)-Alkyl und NR²⁰CO-Pyridyl
R¹⁶ ist Wasserstoff, (C₁-C₄)-Alkyl, Phenyl;
R¹⁷ ist Wasserstoff, Halogen, (C₁-C₄)-Alkyl, NO₂, NH₂;
R²⁰ ist Wasserstoff, (C₁-C₄)-Alkyl, Benzyl;
R²¹ ist Wasserstoff, C(O)-O-(C₁-C₅)-Alkyl;
Z ist -R¹⁴-N-R²²
R²² 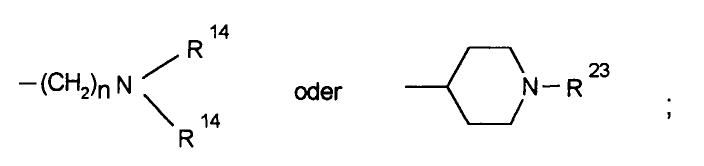
R²³ (C₁-C₄)-Alkyl, 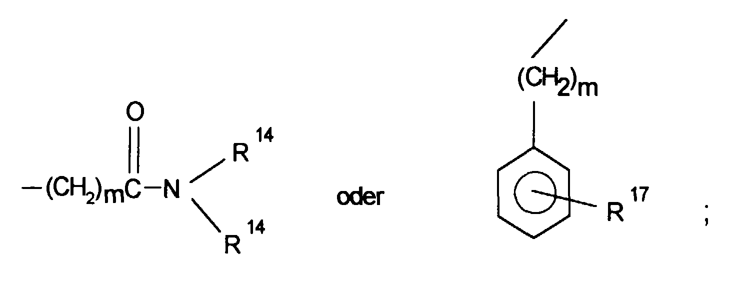
sowie deren physiologisch verträglichen Salze.

4. Verwendung nach Anspruch 2 oder 3 eines nicht-peptidischen Bradykininantagonisten der Formel (I), in welcher die Symbole folgende Bedeutung haben:
D ein Rest der allgemeinen Formel (X) 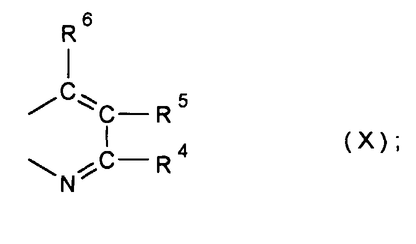
E ein Rest der Formel (XI) 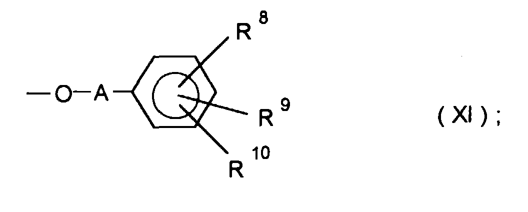
R¹,R² sind gleich oder verschieden uns stehen für Wasserstoff, Halogen oder (C₁-C₄)-Alkyl;
R⁴ Wasserstoff, (C₁-C₄)-Alkyl;
R⁵ Wasserstoff;
R⁶ Wasserstoff;
R⁸,R⁹ sind gleich oder verschieden und stehen für Wasserstoff oder Chlor;
A -CH₂- oder -CH₂-CH₂- ;
R¹⁰ ein Rest der Formel (IX): 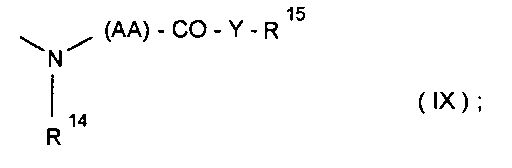
R¹⁴ Wasserstoff, Methyl oder Ethyl;
AA steht für die Aminosäure Glycin;
Y
1. (C₂-C₅)-Alkendiyl,
2. (C₂-C₄)-Alkandiyl,
3. -(CH₂)ₚ-Tₒ-(CH₂)_{q}- ;
T steht für 0 oder S;
o ist eine Zahl 0 oder 1;
p,q sind gleich oder verschieden und bedeuten eine Zahl von 0-2;
R¹⁵
1. Wasserstoff
2. (C₁-C₃)-Alkyl,
3. Phenyl,
4. (C₅-C₉)-Heteroaryl,
wobei 3. und 4. gegebenenfalls mit einer, zwei oder drei Gruppen substituiert sein können, wie Halogen, NO₂, (C₁-C₃)-Alkyl, (C₁-C₃)-Alkyl, in dem die H-Atome teilweise oder vollständig durch Halogen ersetzt sind, (C₁-C₃)-Alkoxy, NR²⁰R²¹, NR²⁰CO-(C₁-C₃)-Alkyl und NR²⁰CO-Pyridyl;
R²⁰ ist Wasserstoff, (C₁-C₄)-Alkyl, Benzyl;
R²¹ ist Wasserstoff, C(O)-O-(C₁-C₅)-Alkyl;
sowie deren physiologisch verträglichen Salze.
